Europäisches Patentamt

European Patent Office

Office européen des brevets

(11) Veröffentlichungsnummer : **0 014 349**
**B1**

(12) **EUROPÄISCHE PATENTSCHRIFT**

(45) Veröffentlichungstag der Patentschrift :
07.04.82

(51) Int. Cl.³ : **G 01 N 33/86, G 01 N 33/96**

(21) Anmeldenummer : **80100222.1**

(22) Anmeldetag : **17.01.80**

(54) Kontrollreagens für die Bestimmung der Heparinaktivität.

(30) Priorität : 31.01.79 DE 2903701

(43) Veröffentlichungstag der Anmeldung :
20.08.80 (Patentblatt 80/17)

(45) Bekanntmachung des Hinweises auf die Patenterteilung : 07.04.82 Patentblatt 82/14

(84) Benannte Vertragsstaaten :
AT BE CH DE FR GB IT LU NL SE

(56) Entgegenhaltungen :
EP - A - 0 004 271
FR - A - 2 353 063
US - A - 4 067 777

(73) Patentinhaber : BOEHRINGER MANNHEIM GMBH
Sandhofer Strasse 112-132 Postfach 31 01 20
D-6800 Mannheim 31-Waldhof (DE)

(72) Erfinder : Bartl, Knut, Dr.
An der Leiten 11
D-8132 Tutzing (DE)
Erfinder : Ziegenhorn, Joachim, Dr.
Ina-Seidel-Weg 1
D-8130 Starnberg (DE)
Erfinder : Wunderwald, Peter, Dr.
Schulstrasse 6
D-8121 Haunshofen (DE)
Erfinder : Beaucamp, Klaus, Dr.
Von-Kühlmann-Strasse 15
D-8132 Tutzing (DE)
Erfinder : Lill, Helmut, Dr.
Zugspitzstrasse 24
D-8121 Wielenbach (DE)

(74) Vertreter : Weickmann, Heinrich, Dipl.-Ing Patentanwälte Dipl.Ing.H.Weickmann et al
Dipl.Phys.Dr.K.Fincke Dipl.Ing.F.A.Weickmann
Dipl.Chem.B.Huber, Dr.-Ing.H.Liska Möhlstrasse 22
D-8000 München 86 (DE)

## Kontrollreagens für die Bestimmung der Heparinaktivität

Die Erfindung betrifft ein Kontrollreagens für die Bestimmung der Heparinaktivität (Heparinstandard), welches zur Eichung bei der Bestimmung des biologisch aktiven Heparins im Plasma geeignet ist.

Die Bestimmung der biologischen Aktivität des Heparins stellt einen wichtigen Parameter für die Verlaufskontrolle einer Heparintherapie dar. Nach dem in der deutschen Offenlegungsschrift 28 12 943 beschriebenen Verfahren erfolgt sie über die Messung der Hemmwirkung des Heparin/Antithrombin-III-(AT III)-Komplexes auf Thrombin. Für diese kommerzielle Bestimmungsmethode ist die Durchführbarkeit an den verschiedenen Analysenautomaten, die dem Untersuchungslabor zur Verfügung stehen, von Bedeutung. Aus verschiedenen Gründen ist es vorteilhaft, die Auswertung des Tests über eine Standard-Eichkurve vorzunehmen :

1. Einige an den Automaten vorgegebene und nicht veränderbare Meßparameter, wie z.B. Inkubationszeit, Dosiervolumina führen dazu, daß für die gleiche Probe unterschiedliche Hemmwerte bzw. Thrombinrestaktivitäten an den einzelnen Automaten erhalten werden. Daher wäre es notwendig, für die diversen Automatenmethoden und die manuelle Methode jeweils unterschiedliche therapeutische Bereiche anzugeben, was in der Praxis unpraktikabel ist.

2. Die Größe der Thrombinrestaktivität ist zum Teil von dem Thrombin-Ausgangswert abhängig. Dieser kann produktionsbedingt schwanken, wodurch sich wiederum Störungen bei der Richtigkeit der Messung ergeben würden.

Da die exakte Einstellung der Heparinkonzentration äußerst wichtig ist (Gefahr der Thrombose einerseits, von Verblutungen andererseits), können auch geringe Analysenfehler obengenannter Art zu schwerwiegenden Folgen, bis zum Tod des Patienten, führen. Der Einsatz von Standards ist daher geeignet, Analysenfehler dieser Art zu verhindern.

Die bekannten Methoden zur Kontrolle der Heparintherapie bestimmen nur die Konzentration des Heparins im Plasma in Gegenwart eines Überschusses an AT III. Bei der Herstellung der hierbei eingesetzten Standards werden daher ausschließlich wäßrige Heparinlösungen eingesetzt, die vor Testdurchführung einem standardisierten Normalplasma in Gegenwart eines Überschusses an AT III zugefügt werden.

Weitere bekannte kommerziell erhältliche Eichplasmen zur Aktivitätsbestimmung von Gerinnungsparametern enthalten nur AT III (ohne Heparin), womit sie für den Einsatz als Standards in Testsystemen, welche die Hemmwirkung des Heparin/AT III-Komplexes auf Thrombin bestimmen, nicht geeignet sind. Außerdem erfüllen derartige Kontrollplasmen aus verschiedenen Gründen nicht die Anforderungen an die in dieses System einzusetzenden Standards : Die Haltbarkeit von Kontrollplasmen ist nämlich nach Rekonstitution auf 1 bis 2 Tage bei 4 °C beschränkt. Ein weiteres Problem beim Einsatz von Kontrollplasmen zur Kalibrierung liegt darin, daß hier für den Sollwert ein relativ großer Streubereich (2s-Bereich) angegeben wird. Zur Kalibrierung ist jedoch insbesondere bei der Heparin-Aktivitätsbestimmung eine exakte Sollwertangabe mit einem sehr geringen Streubereich nötig, da schon geringe Meßfehler aufgrund falscher Kalibrierung in der Klinik zu schwerwiegenden Folgen führen können.

Der Erfindung liegt daher die Aufgabe zugrunde, ein stabiles Kontrollreagens (Heparin-Standard) zu schaffen, welches zum Einsatz bei der Kontrolle einer Heparintherapie über die Bestimmung der biologischen Aktivität des Heparins geeignet ist. Da sich das Reagens zur Erstellung einer Eichkurve eignen sollte, genügt es nicht, einen Streubereich für die Heparinaktivität einzuhalten. Vielmehr soll der einmal eingestellte und ermittelte Sollwert über den angegebenen Haltbarkeitszeitraum exakt konstant bleiben, d.h. die Lagerfähigkeit muß hohen Ansprüchen genügen.

Gelöst wird diese Aufgabe erfindungsgemäß durch ein Kontrollreagens für die Bestimmung der Heparinaktivität, welches dadurch gekennzeichnet ist, daß es

20 bis 1 500 USP Heparin,
2 000 bis 30 000 IU Antithrombin III,
0,15 bis 1,5 mMol Serumalbumin,
0,005 bis 0,05 Mol Äthylendiamintetraessigsäure-natriumsalz,
0 bis 0,4 Mol Saccharose,
2 000 bis 20 000 U Aprotinin und
Puffersubstanz, pH 6,5 bis 7,5

in gelöster oder lyophilisierter Form, bezogen auf die zur Herstellung von 1 l Lösung erforderliche Menge, enthält oder daraus besteht.

Durch den Einsatz bestimmter Mengen von Heparin und/oder AT III wird ein Hemmwert eingestellt, der einer bestimmten Heparinmenge im Normalplasma entspricht.

Überraschenderweise ist es erfindungsgemäß möglich, einen sehr stabilen antithrombin-III-haltigen Heparinstandard zu erhalten. Zwar wird das Antithrombin III als « relativ stabil » (Methods of Enzymology, Vol. 45, B, Ed. Lorand, 653 (1976)) beschrieben, jedoch genügt diese Stabilität bei weitem nicht den obigen Anforderungen hinsichtlich Haltbarkeit von Standards, die zur Kontrolle der Heparintherapie geeignet sind. Der erfindungsgemäße Standard aber ist z.B. mit 40 % Hemmung, was 0,3 USP Heparin/ml Normalplasma entspricht, selbst nach einer dreiwöchigen Belastung bei 33 °C noch vier Wochen bei −20 °C und anschließend 7 Tage bei 4 °C stabil, d.h. sein Hemmwert ändert sich um weniger als 5 % vom Ausgangswert.

Weitere Stabilitätsdaten von erfindungsgemäßen Standards mit verschiedenen Hemmwerten sind in der Tabelle der Beispiele angegeben.

Da bei dem Testsystem gemäß deutscher Offenlegungsschrift 28 12 943, für welches der erfindungsgemäße Standard in erster Linie bestimmt ist, die Aktivität des Heparins in %-Hemm- bzw. -Restaktivität, bezogen auf eingesetztes Thrombin, bestimmt wird, werden bei dem Standard nicht wie bei den bekannten Standard- bzw. Kontrollplasmen die Konzentrationen der einzelnen Meßparameter Heparin und AT III angegeben, sondern die Heparinmenge, die der eingestellten Thrombinrestaktivität in gepooltem Normalplasma (normaler AT III-Gehalt) entspricht. Diese Restaktivität kann entweder durch Änderung der Heparin- und/oder AT III-Konzentration erreicht werden. Da dieser Hemmwert jedoch schon geringe Änderungen von Heparin und/oder AT III außerordentlich empfindlich anzeigt, ist die lange Stabilität um so überraschender. Als AT III wird zweckmäßig ein nach bekannten Verfahren gereinigtes, für Heparin hochaffines AT III-Lyophilisat eingesetzt. Als Heparinquelle können im wesentlichen die kommerziell erhältlichen Heparinpräparate benützt werden.

Geeignete Puffersubstanzen sind solche, welche im angegebenen pH-Bereich wirksam sind. Die Konzentration liegt zweckmäßig zwischen 1 und 300 mMol/l, vorzugsweise 1 bis 10 mMol/l. Bevorzugte Puffersubstanzen sind Tris, Citrat und Phosphat.

Die überraschend hohe Stabilität des erfindungsgemäßen Standards wird nur erzielt, wenn alle Bestandteile in den angegebenen Mengen vorhanden sind. In einer bevorzugten Zusammensetzung enthält das Reagens, bezogen auf 1 l Lösung

0,8 bis 1,0 mMol Rinderserumalbumin,
0,009 bis 0,011 Mol Äthylendiamintetraessigsäure-natriumsalz,
0,03 bis 0,05 Mol Saccharose,
8 000 bis 12 000 U Aprotinin und
Puffersubstanz, pH 6,9 bis 7,1.

Das erfindungsgemäße Reagens ermöglicht es, mit dem Testsystem gemäß deutscher Offenlegungsschrift 28 12 943 die Bestimmung der biologischen Aktivität des Heparins in praktikabler Weise sowohl manuell als auch an den diversen Analysenautomaten durchzuführen. Anders als bei den bekannten Standards zur Konzentrationsbestimmung von Heparin ist es bei dem erfindungsgemäßen Standard zur Aktivitätsbestimmung des Heparins nicht mehr erforderlich, jeweils das zur Therapie eingesetzte Heparinpräparat infolge unterschiedlicher Aktivitäten auch zur Herstellung des Standards einzusetzen. Die Praktikabilität der Testdurchführung in der Routine wird dadurch wesentlich verbessert.

Beispiele 1 bis 4

Vier erfindungsgemäße Standards enthielten je 0,88 mMol/l Rinderserumalbumin, 0,01 Mol/l Äthylendiamintetraessigsäure-natriumsalz und 0,04 Mol/l Saccharose, 10 000 U/l Aprotinin und 2 mMol/l Tris-Puffer, pH 7,0. Die zur Erzielung der in der nachstehenden Tabelle angegebenen unterschiedlichen Hemmwerte eingesetzten Mengen an Heparin und Antithrombin III zeigt die Tabelle. Die zur Herstellung des gewünschten Hemmwertes benötigten Mengen dieser Substanzen hängen entsprechend ihrer Aktivität auch von den Parametern des Testsystems ab, für welches sie bestimmt sind, z.B. dem Thrombinausgangswert.

Tabelle

| | Thrombinrestaktivität % | | | |
| | Beispiel 1 | Beispiel 2 | Beispiel 3 | Beispiel 4 |
|---|---|---|---|---|
| AT III | 10 U/ml | 10 U/ml | 10 U/ml | 10 U/ml |
| Heparin | 0,06 USP/ml | 0,28 USP/ml | 0,5 USP/ml | 0,8 USP/ml |
| Lyophilisat Ausgangswert | 86,6 | 59,2 | 40,6 | 27,2 |
| Lyophilisat gelöst 7 Tage + 4 °C | 87,0 | 61,1 | 42,0 | 28,3 |
| Lyophilisat 3 Wochen 33 °C gelagert | 86,6 | 59,1 | 40,1 | 24,6 |
| gelagertes Lyophilisat, gelöst 7 Tage + 4 °C | 86,6 | 61,9 | 42,2 | 26,6 |

Tabelle (Fortsetzung)

| | Thrombinrestaktivität % | | | |
| | Beispiel 1 | Beispiel 2 | Beispiel 3 | Beispiel 4 |
|---|---|---|---|---|
| gelagertes Lyophilisat, gelöst 28 Tage − 20 °C | 86,7 | 59,5 | 40,0 | 25,1 |
| gelagertes Lyophilisat 28 Tage − 20 °C 7 Tage + 4 °C | 87,3 | 60,9 | 42,9 | 27,0 |

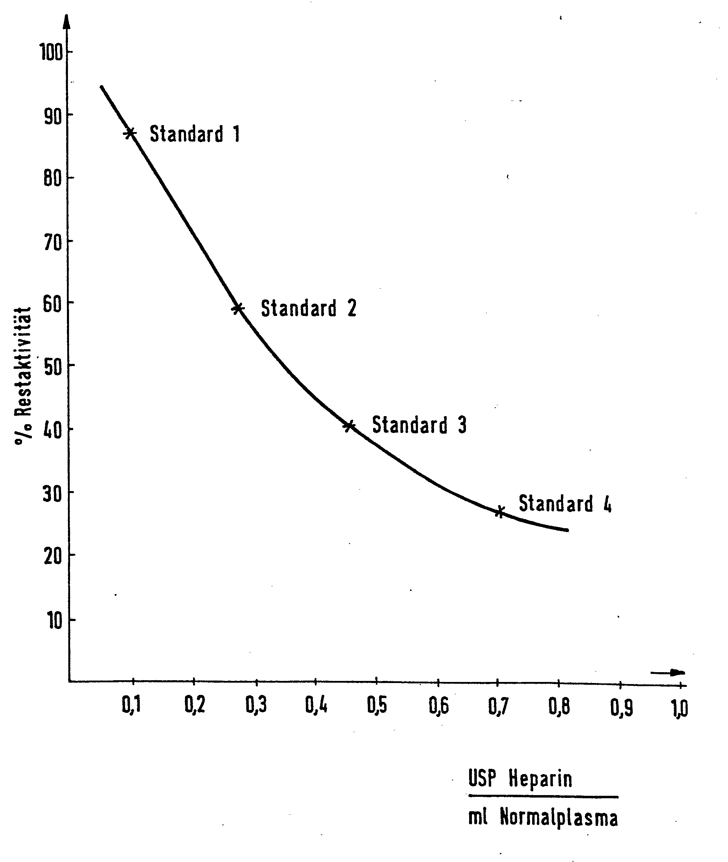

Die mit den Standards der Beispiele 1 bis 4 erhaltene Standardkurve zeigt die beigefügte Zeichnung.

**Ansprüche**

1. Kontrollreagens für die Bestimmung der Heparinaktivität, dadurch gekennzeichnet, daß es

20 bis 1 500 USP Heparin,
2 000 bis 30 000 IU Antithrombin III,
0,15 bis 1,5 mMol Serumalbumin
0,005 bis 0,05 Mol Äthylendiamintetraessigsäure-natriumsalz,
0 bis 0,4 Mol Saccharose,
2 000 bis 20 000 U Aprotinin und
Puffersubstanz, pH 6,5 bis 7,5,

in gelöster oder lyophilisierter Form, bezogen auf die zur Herstellung von 1 l Lösung erforderliche Menge, enthält oder daraus besteht.

2. Reagens nach Anspruch 1, dadurch gekennzeichnet, daß es

0,8 bis 1,0 mMol Rinderserumalbumin,
0,009 bis 0,011 Mol Äthylendiamintetraessigsäure-natriumsalz,
0,03 bis 0,05 Mol Saccharose,
8 000 bis 12 000 U Aprotinin und
Puffersubstanz, pH 6,9 bis 7,1,

enthält.

**Claims**

1. Control reagent for the determination of heparin activity, characterized in that it consists of or contains

20 to 1 500 USP heparin,
2 000 to 30 000 IU antithrombin III,
0.15 to 1.5 mMol serum albumin,
0.005 to 0.05 mMol ethylenediamintetraacetic acid-sodium salt,
0 to 0.4 Mol saccharose,
2 000 to 20 000 U aprotinine and
buffer substance, pH 6.5 to 7.5,

in dissolved or lyophilised form, based on the amount necessary to prepare 1 litre of solution.

2. Reagent according to claim 1, characterized in that it contains

0.8 to 1.0 mMol beef serum albumin,
0.009 to 0.011 Mol ethylenediamintetraacetic acid-sodium salt,
0.03 to 0.05 mol saccharose,
8 000 to 12 000 U aprotinine and buffer substance, pH 6.9 to 7.1.

**Revendications**

1. Réactif de contrôle pour la détermination de l'activité de l'héparine, caractérisé en ce qu'il

4

contient ou consiste en :

20 à 1 500 USP d'héparine,
2 000 à 30 000 UI d'antithrombine III,
0,15 à 1,5 mmole d'albumine du sérum,
0,005 à 0,05 mole de sel de sodium de l'acide éthylènediaminetétracétique,
0 à 0,4 mole de saccharose,
2 000 à 20 000 U d'aprotinine et
une substance tampon pH 6,5 à 7,5,

à l'état dissous ou lyophilisé par rapport à la quantité nécessaire à la préparation d'1 litre de solution.
2. Réactif selon la revendication 1, caractérisé en ce qu'il contient :

0,8 à 1,0 mmole d'albumine de sérum de bovins,
0,009 à 0,011 mole de sel de sodium de l'acide éthylène-diaminetétracétique,
0,03 à 0,05 mole de saccharose,
8 000 à 12 000 U d'aprotinine et
une substance tampon, pH 6,9 à 7,1.